# EUROPEAN PATENT SPECIFICATION

(11) **EP 1 620 469 B1**
(45) Date of publication and mention of the grant of the patent: **10.04.2013**
(21) Application number: 04730430.8
(22) Date of filing: 30.04.2004
(51) Int. Cl.: A61K 31/716, A61K 36/899, A61K 36/8998, C08B 37/00

(54) **IMPROVED EXTRACTION AND PURIFICATION METHOD FOR CEREAL BETA (1-3) BETA (1-4) GLUCAN**
VERFAHREN FÜR DIE VERBESSERTE EXTRAKTION UND REINIGUNG VON BETA (1-3) BETA (1-4) GLUKAN AUS GETREIDEN
PROCEDE AMELIORE D'EXTRACTION ET DE PURIFICATION DE CEREALES BETA (1-3) BETA (1-4) GLUCANE

(30) Priority: 02.05.2003 US 467146 P; 10.06.2003 US 477048 P
(43) Date of publication of application: 01.02.2006
(60) Divisional application: 12170299.7
(73) Proprietor: Ceapro Inc., Edmonton, AB T5J 4P6 (CA)
(72) Inventor: REDMOND, Mark, J., Alberta T6G 1B3 (CA); FIELDER, David, A., Edmonton, Alberta T6A 2S6 (CA)
(74) Representative: Harding, Charles Thomas
(86) International application number: PCT/CA2004/000666
(87) International publication number: WO 2004/096862

(56) References cited:
- WO-A-02/02645
- WO-A-02/43719
- US-A- 5 518 710
- US-A- 5 980 918
- US-A1- 2002 016 454
- US-B1- 6 284 886
- WESTERLUND E ET AL: "ISOLATION AND CHEMICAL CHARACTERIZATION OF WATER-SOLUBLE MIXED-LINKED B-GLUCANS AND ARABINOXYLANS IN OAT MILLING FRACTIONS" CARBOHYDRATE POLYMERS, APPLIED SCIENCE PUBLISHERS, LTD. BARKING, GB, vol. 20, no. 2, 1993, pages 115-123, XP000343989 ISSN: 0144-8617
- WIKSTROM KATARINA ET AL: "Rheological studies of water-soluble (1-3),(1-4)-beta-D-glucans from milling fractions of oat" JOURNAL OF FOOD SCIENCE, vol. 59, no. 5, 1994, pages 1077-1080, XP001194837 ISSN: 0022-1147
- WOOD P J ET AL: "LARGE-SCALE PREPARATION AND PROPERTIES OF OAT FRACTIONS ENRICHED IN (1 3(1 4)-BETA-D-GLUCAN" CEREAL CHEMISTRY, AMERICAN ASSOCIATION OF CEREAL CHEMISTS. MINNEAPOLIS, US, vol. 66, no. 2, 1989, pages 97-103, XP001194802 ISSN: 0009-0352

## Description

### TECHNICAL FIELD

The present invention relates generally to cereal β-glucans. More particularly, the invention relates to methods for extracting and isolating highly purified oat β (1-3) β (1-4) glucan. The present description also particularly relates to compositions comprising a β (1-3) β (1-4) glucan and a freezing point depressant.

### BACKGROUND OF THE INVENTION

Gums are either hydrophobic or hydrophilic substances of molecular weight ranging from 10,000 to 50,000,000 Daltons, which in an appropriate solvent produce gels or highly viscous suspensions or solutions at low dry substance content. Gums commonly used in food, medicine, and industrial products include starches, cellulose derivatives, pullulan, agar, aloe, gellan, guar gum, locust bean gum, pectin, algin, carrageenan, xanthan, β-glucan, and gum arabic (see Whistler, R.L. (1993) Industrial Gums: Polysaccharides and their derivatives Eds. Whistler R.L. and BeMiller J.N. (Academic Press) p 2).

Glucans are homopolysaccharides consisting only of glucose. However, since it is possible to link the glucose molecules in different stereochemical configurations, glucans are a diverse group of compounds with differing chemical, physical, and functional properties.

Chemical structures of polysaccharides are of prime importance in determining their properties. This can be appreciated by comparing the properties of some common homoglucans. For example, cellulose, a β (1-4)-D-glucan, is water insoluble and highly crystalline compared to other polysaccharides. Amylose, an α (1-4)-D-glucan, is sparingly soluble in water, crystallizes less well than cellulose, and can form rigid thermo-reversible gels. Dextran, a (1-6)-α-glucan, with a small degree of branching, is extremely water soluble and non-gel forming. (See Dea, I.C.M. in (1993) Industrial Gums: Polysaccharides and their derivatives Eds. Whistler R.L. and BeMiller J.N. (Academic Press) p 21).

Oat β (1-3) β (1-4) glucan is classified as a viscous gum, (see Wood, P.J. (1993) Oat Bran Ed P.J. Wood (American Association of Cereal Chemists, Inc. St. Paul, MN)). Cereal β (1-3) β (1-4)-glucans are structural polysaccharides present in the cell walls of cereals, such as barley and oat, among others.

Oat β (1-3) β (1-4) glucan is recognised by the U.S. FDA as an agent that may aid in the prevention of heart disease. In 1997, the FDA allowed oat products to make a health claim. It is important to note that no other β-glucan source, yeast, fungal, bacterial, or cereal is recognised as having these effects. Oat P (1-3) β (1-4)-glucans are therefore distinct.

Unmodified oat β (1-3) β (1-4) glucan forms highly viscous solutions in water at concentrations >0.75%. At concentrations >1.2% the solutions have the consistency of a thick hydrogel.

Glucans of significantly different molecular structure and with different physical and chemical properties compared to oat are found in yeast, fungi, and certain bacteria and genetically engineered bacteria. For example, gellan, polymeric (1-3) β-D-glucopyranosyl [β (1-3)-glucan] produced in *Alcaligenes faecalis* is found in Curdlan (Takeda Chemical Ind. Ltd.), P (1-3) α (1-6) glucopyranoside produced in *Aureobasidium pullulans* is found in pullulan, and β (1-3) β (1-6) glucopyranoside is found in yeast.

The molecular weight of the glucans varies with source. Table 1 shows the average molecular weight of typical gums.

**Table 1: Typical Molecular Weight Range of Common Gums**

| **GUM** | **AVERAGE MOLECULAR WEIGHT** |
|---|---|
| Oat β (1-3) β (1-4) glucan | 500,000 - 1,000,000 . |
| Pullulan | 50,000 - 100,000 |
| Curdlan | ~500,000 |
| Methyl cellulose | 10,000 - 200,000 |
| Carrageenan | 4,500,000 |
| Xanthan | 15,000,000 - 50,000,000 |
| Sodium alginate | 10,000 - 18,000,000 |

The viscosity of a 1% solution of different polysaccharide gum solutions varies with origin and chemical nature. Table 2 shows the viscosity of 1% solutions of typical gums.

**TABLE 2: Typical Viscosity Ranges of 1% Solutions of Common Gums, Measured at 25°C**

| **GUMS** | **1% SOLUTION VISCOSITY, cP** |
|---|---|
| Oat β (1-3) β (1-4) glucan | 500-1500 |
| Pullulan | 2 |
| Gum Arabic | 1-5 |
| Methyl cellulose | 200 |
| Tamarind gum | 100-200 |
| Guar gum | 2,000-3,000 |
| Locust bean gum | 2,000-3,000 |
| Xanthan | 2,000-3,000 |
| Sodium alginate | 200-700 |

The solubility properties of glucans differ according to their source. For example, cereal β (1-3) β (1-4) glucans are normally soluble in aqueous solvents, whereas yeast *(Saccharomyces cerevisiae)* β (1-3) β *(1-6)-glucans* are insoluble in aqueous solvents. Soluble-glucans are desirable. Yeast β-glucan has been solubilized by the addition of phosphate groups (see Williams et al. Immunopharmacol. 22: 139-156 (1991). Jamas et al. (U.S. Patent No. 5,622,939) describes methods to extract soluble β (1-3) β (1-6) glucan from *Saccharomyces cerevisiae.* The method described is complex involving acid hydrolysis, base hydrolysis and the extensive use of centrifugation and ultrafiltration. No details are provided as to the stability of the solubilized yeast β (1-3) β (1-6) glucan.

A number of prior art references disclose methods of preparing-glucans and liquid-glucan compositions from cereals. Among these prior art references are the following:
Beer, et al., Extraction of Oat Gum from Oat Bran: Effects of Process on Yield, Molecular Weight Distribution, Viscosity and (1-3) (1-4) beta-D-Glucan Content of the Gum, Cereal Chemistry 73(1): 58-62 (1996). This reference describes the use of alcohols in an amount equal to or greater than 50% (v/v) to achieve precipitation. The purity of the recovered glucans was reported to be between 22 and 63%.
Wood et al., Large Scale Preparation and Properties of Oat Fractions Enriched in (1-3)(1-4) beta-D-Glucan, Cereal Chemistry 66(2): 97-103 (1989). This reference describes the use of alcohols in an amount equal to or greater than 50% (v/v) to achieve precipitation of glucans.
U.S. Patent No. 6,323,338 discloses a method of isolating oat β-glucan as an enriched skin from an extract of oat bran. The disclosed method does not utilize low concentrations of short-chain alcohols for the precipitation of the glucan.
Redmond (U.S. Patent No. 6,284,886) discloses compositions of cereal β (1-3) β (1-4) glucans, and methods of producing these compositions. The disclosed compositions meet the strict requirements of the cosmetics industry, in terms of their viscosity, shear strength, and moisture-enhancing properties. No method for the extraction or purification of β (1-3) β (1-4) glucan is described.

### SUMMARY OF THE INVENTION

The present invention is summarised in the following numbered paragraphs:-
1. A method of isolating a β (1-3) β (1-4) glucan from a milled cereal grain or a milled part of the cereal grain, comprising:
   (i) extracting the milled cereal grain or the milled part of the cereal grain with an alkaline solution having a value of pH of between 9 to 10 for a period of time of about 15 to about 45 minutes to produce an extract containing at least about 0.4 weight percent β (1-3) β (1-4) glucan;
   (ii) removing insoluble material, and removing particulate material having a particle size of greater than about 0.2 µm from said extract to produce a purified extract;
   (iii) adding from about 10% to about 25% (vol/vol) of a C₁-C₄ alcohol to the purified extract to precipitate the β (1-3) β (1-4) glucan, and
   (iv) isolating the β (1-3) β (1-4) glucan.
2. The method of paragraph 1, wherein, in said step of adding (step iii), about 10% to about 20% (vol/vol) of an alcohol selected from the group consisting of methanol, ethanol and isopropanol, is used to precipitate the β (1-3) β (1-4) glucan from said purified extract.
3. The method of paragraph 2, wherein about 10% to about 20% (vol/vol) of ethanol is used to precipitate the β (1-3) β (1-4) glucan from said purified extract.
4. The method of paragraph 1, wherein, said step of removing particulate material further comprises
   one, or more than one step of adding a flocculant, a coagulant or both a flocculant and a coagulant to said extract to coagulate particulate material having a particle size of greater than about 0.2 µm, and removing coagulated material from said extract;
   digesting starch material in said extract, and
   filtering out particulate material having a particle size of greater than 0.2 µm from said extract to produce the purified extract.
5. The method of paragraph 4, wherein, in said step of digesting, said starch material is digested with an enzyme.
6. The method of paragraph 5, wherein prior to digesting said starch material, said alkaline solution is neutralized.
7. The method of paragraph 6, wherein following the digestion of said starch material, said enzyme is inactivated.
8. The method of paragraph 7, wherein said enzyme is inactivated by acidifying the neutralized solution.
9. The method of paragraph 5, wherein said enzyme is an amylase.
10. The method of paragraph 9, wherein said amylase does not require a calcium cofactor.
11. The method of paragraph 1, wherein the cereal is selected from the group consisting of a cultivar of barley, a cultivar of oat, a cultivar of wheat, a cultivar of rye, a cultivar of sorghum, a cultivar of millet, and a cultivar of corn.
12. The method of paragraph 1, wherein said step of adding (step iii) is conducted at a temperature of from about 1°C to about 10°C.
13. The method of paragraph 1, further comprising one, or more than one step of dissolving the isolated β (1-3) β (1-4) glucan in an aqueous solution, precipitating the β (1-3) β (1-4) glucan by adding between 10% to 25% (vol/vol) of the C₁-C₄ alcohol to the aqueous solution, and isolating the β (1-3) β (1-4) glucan.
14. The method of paragraph 4, wherein the flocculant is selected from the group consisting of a polyacrylamide, a quaternary acrylate salt and a natural flocculant macromolecule, and the coagulant is selected from the group consisting of alum, lime, ferric chloride, ferrous sulfate, an organic polymer and a synthetic polyelectrolyte with anionic or cationic functional groups.
15. The method of paragraph 1, wherein about 15% to about 17% (vol/vol) of the C₁-C₄ alcohol is added to the purified extract in step (iii).
16. The method of paragraph 1, wherein the milled cereal grain or the milled part of the cereal grain is extracted with an alkaline solution having a value of pH of about 9.25 to about 9.75.
17. The method according to paragraph 4, wherein the step of filtering out material having a particle size of greater than 0.2 µm from said extract is conducted using a filter coated with a pre-coat of a filter aid having a porosity of 0.2 µm.

The present invention relates generally to cereal β-glucans. More particularly, the invention relates to methods for extracting and isolating highly purified oat β (1-3) β (1-4) glucan. The present description also particularly relates to compositions comprising a β (1-3) β (1-4) glucan and a freezing point depressant.

In one aspect, herein is described a method for the extraction and purification of a cereal β-glucan. The cereal β-glucan is derived from a cereal grain or a part of the cereal grain.

In particular, the present description provides a method of isolating a β (1-3) β (1-4) glucan from a milled cereal grain or a milled part of the cereal grain, comprising:
(i) extracting the milled cereal grain or the milled part of the cereal grain with an alkaline solution to produce an extract containing at least about 0.4 weight percent β (1-3) β (1-4) glucan;
(ii) removing insoluble material, and removing particulate material having a particle size of greater than about 0.2 µm from the extract to produce a purified extract;
(iii) adding from about 10% to about 25% (w/w) of a C₁-C₄ alcohol to the purified extract to precipitate the β (1-3) β (1-4) glucan, and
(iv) isolating the β(1-3) β(1-4) glucan.

In an example of the above-defined method, about 10% to about 20% (w/w) of an alcohol selected from the group consisting of methanol, ethanol and isopropanol is used to precipitate the β (1-3) β (1-4) glucan from the filtrate. Preferably, about 10% to about 20% (w/w) of ethanol is used to precipitate the β (1-3) β (1-4) glucan.

In an example of the methods described above, the step of removing particulate material comprises:
one, or more than one step of adding a flocculant, a coagulant or both a flocculant and a coagulant to the extract to coagulate particulate material having a particle size of greater than about 0.2 µm, and removing coagulated material from the extract;
digesting starch material in the extract, and
filtering out particulate material having a particle size of greater than about 0.2 µm from the extract to produce a purified extract.

In an example of the just described method, the starch material is digested with an enzyme, such as an amylase. More particularly, the enzyme is digested with an amylase that does not require a calcium cofactor. In another example, the alkaline extract is neutralized before the starch material is digested. In a further example, the enzyme is inactivated following the digestion of the starch material, by, for example, acidifying the alkaline extract containing the digested starch material.

The present description also relates to the above-defined methods, wherein the cereal is selected from the group consisting of a cultivar of barley, a cultivar of oat, a cultivar of wheat, a cultivar of rye, a cultivar of sorghum, a cultivar of millet, a cultivar of corn, and a mixture thereof

The present description is also directed to the methods described above, wherein the cereal grain or the part of the cereal grain extracted in step (i) is in the form of a coarsely-milled flour or a finely-milled flour.

In other examples of the above-described methods, the pH value of the alkaline solution is from about 9.00 to about 10.00, from about 9.25 to about 9.75, or from about 9.30 to about 9.50. In another example, the step of extracting (step i) is carried out over a period of about 15 to about 45 minutes. ,

In a further example of the above-defined methods, the precipitation step is conducted at a temperature of from about 1°C to about 10°C, or from about 1°C to about 5°C. In an even further example, the alcohol used to conduct the precipitation step is cooled to a temperature of at least about -20°C before being added to the β(1-3) β(1-4) glucan solution.

The methods defined above may further comprise one, or more than one step of dissolving the isolated β (1-3) β (1-4) glucan from step (iv) in an aqueous solution, precipitating the (1-3) β (1-4) glucan by adding about 10% to about 25% (w/w) of the C₁-C₄ alcohol to the aqueous solution, and isolating the β (1-3) β (1-4) glucan.

The present description also provides a method of isolating a β (1-3) β (1-4) glucan from a milled cereal grain or a milled part of the cereal grain, comprising:
(i) extracting the milled cereal grain or the milled part of the cereal grain with an alkaline solution to produce an extract comprising at least about 0.4 weight percent β (1-3) β (1-4) glucan;
(ii) removing insoluble material, and removing particulate material having a particle size of greater than about 0.2 µm from the extract to produce a purified extract, wherein the step of removing particulate material comprises:
   one, or more than one step of adding a flocculant, a coagulant or both a flocculant and a coagulant to the extract to coagulate particulate material having a particle size of greater than about 0.2 µm, and removing coagulated material from the extract;
   enzymatically digesting starch material in the extract, and
   filtering out particulate material having a particle size of greater than about 0.2 µm from the extract to produce the purified extract;
(iii) adding about 10% to about 25% (w/w) of a C₁-C₄ alcohol to the purified extract to precipitate the β(1-3) β(1-4) glucan, and
(iv) isolating the β(1-3) β(1-4) glucan.

In a second aspect, the present description provides a cereal β-glucan composition having a purity of at least about 75%, and containing less than10% ash impurities, less than 10% protein impurities, and less than 5% lipid impurities. More particularly, the present description relates to a cereal β-glucan composition having a purity of at least about 92%, and containing less than 3.5% ash impurities, less than 3.5 % protein impurities, and less than 1% lipid impurities. The cereal β-glucan composition can also have a clarity value of from about 5 to about 100 NTU.

In a third aspect, the present description provides a composition comprising a β (1-3) β (1-4) glucan and from about 1% to about 40% by weight of a freezing point depressant.

In examples of the above-defined composition, the β (1-3) β (1-4) is present in an amount of from about 1.2% to about 1.6% or from about 1.2% to about 1.3% by weight. In another example, the freezing point depressant is selected from the group consisting of glycerol, propylene glycol, butylene glycol and pentylene glycol.

In a further example of the above-described composition, the β (1-3) β (1-4) glucan is a β (1-3) β (1-4) glucan composition having a purity of at least about 75%, and containing less than 10% ash impurities, less than 10% protein impurities, and less than 5% lipid impurities. More particularly, the β -glucan composition has a purity of at least about 92%, and contains less than 3.5% ash impurities, less than 3.5 % protein impurities, and less than 1% lipid impurities. The P (1-3) β (1-4) glucan composition may also have a clarity of about 5 to about 100 NTU. More particularly, the β (1-3) β (1-4) glucan used in the composition of the fourth aspect of the present description is prepared according to the above-defined isolation methods.

The freezing point depressant prevents appreciable gelling of 0 (1-3) P (1-4) glucan compositions during storage or shipping. β (1-3) β (1-4) glucan compositions containing the freezing point depressant are, therefore, advantageous, .from a commercial standpoint, in that they may be used directly after being stored or shipped without any treatment step to make the compositions more fluid.

The purification method of the present description differs from the method disclosed in U.S. Patent No. 6,323,338 in that fine particulate matter is removed, as well as a large proportion of protein (~90%) present in the original cereal grain.

The purification method of the present description allows the use of concentrations of alcohol of less than 50% (w/w), for example, 10-25% aqueous alcoholic solutions to precipitate cereal β-glucan. The capability of using such concentrations of alcohol is surprising in view of prior art purification procedures, which have used 50% ethanol solutions to precipitate cereal β-glucan (see, for example, Wood et al. Large Scale Preparation and Properties of Oat Fractions Enriched in β (1-3) β (1-4) D-glucan Cereal Chem. 66 97-103 (1989)). It is believed that the removal of the particulate matter and most of the protein material, according to the method of the present description reduces the amount of alcohol needed to precipitate the cereal β-glucan from solution.

The use of 10-25% aqueous alcoholic solutions to precipitate the cereal β-glucan is advantageous in that severe dehydration of the cereal β-glucan is avoided, resulting in a cereal β-glucan precipitate that can be easily suspended in water. Furthermore, the use of these relatively lower alcohol concentrations allows the starting cereal grain to be processed in a standard manufacturing plant without the need for explosion proof environmental systems. For example, use of 20% aqueous alcoholic solutions at a final temperature of 7-10°C produces a vapor pressure lower than the Lower Explosion Limit (LEL) of ethanol. Furthermore, the efficiency of the extraction step and the production of intermediate solutions containing cereal β-glucan at a concentration of greater than 0.4% permits processing using relatively small process volumes.

### DESCRIPTION OF PREFERRED EMBODIMENT

The present invention relates generally to cereal β-glucans. More particularly, the invention relates to methods for extracting and isolating highly purified oat β (1-3) β (1-4) glucan. The present description also particularly relates to compositions comprising a β (1-3) β (1-4) glucan and a freezing point depressant.

The practice of the present invention will employ, unless otherwise indicated, conventional methods of chemistry, cereal chemistry, and biochemistry, within the skill of the art. Such techniques are explained fully in the literature. See for example, Industrial Gums: Polysaccharides and their derivatives Eds. Whistler R.L. and BeMiller J.N. (Academic Press), Oats: Chemistry and Technology ed. Webster F.H. (American Association of Cereal Chemists, St. Paul, MN).

As used in this specification and the appended claims, the singular forms "a", "an" and "the" include plural references unless the content clearly indicates otherwise.

### Definitions

In describing the present invention, the following terms will be employed, and are intended to be defined as indicated below.

By "cereal" is meant any of several grains such as, but not limited to, cultivars of barley, oat, wheat, rye, sorghum, millet, and corn.

By "glycan" is meant a polymer of monosaccharides linked together by glycosidic bonds.

By "glucan" is meant a homopolysaccharide consisting only of glucose.

By "cereal β-glucan" is meant a glucan with a β (1-3)-linked glucopyranosyl backbone, or a β (1-4)-linked glucopyranosyl backbone, or a mixed β (1-3) β (1-4)-linked glucopyranosyl backbone, which is derived from a cereal source.

By "β (1=3) β (1-4) glucan" is meant a cereal β-glucan.

By "gum" is meant a plant or microbial polysaccharide or their derivatives, which are dispersible in either cold or hot water to produce viscous mixtures or solutions. Gums may be classified by origin, and include: exudate gums, seaweed gums, seed gums, starch and cellulose derivatives, and microbial gums.

By "compound of interest" is meant any pharmaceutical, medicinal, botanical or therapetic material mixed with a β (1-3) β (1-4) glucan to produce a composition.

By "flocculant" and "coagulant" are meant molecules that can coalesce with suspended solids (fines) to form larger denser particles that can be separated by centrifugation. In particular examples, coagulants are molecules that can bring together suspended particles that are less than 1 µm in size, and flocculants are molecules that can bring together suspended particles that are greater than 1 µm in size.

By "insoluble material" is meant material that is not soluble under the initial alkaline extraction conditions of the method of the invention. Non-limiting examples of such material include fibre, hemicellulose and lignins.

By "particulate material" is meant a solid or colloidal material having a particle size of greater than about 0.2 µm.

By "a milled cereal grain" or "a milled part of the cereal grain" is meant a cereal grain or part of the cereal grain, which has been ground, abraded or chopped into a meal or flour. In a particular example, the milled part of the cereal grain is bran that has been abraded from the cereal grain, and optionally further ground and purified by, for example, air classification or sieving to provide a specific particle profile.

By "effective amount" is meant the amount of the one, or more than one compound of interest necessary to achieve a desired effect, such as a physiological effect, or a stimulatory effect.

By "sequestered" is meant the incorporation, entrapment, or solubilization of hydrophilic compounds, or hydrophobic compounds, for example, small molecular weight hydrophobic compounds, such as essential oils, pharmaceutical, medicinal, and therapeutic agents.

By "freezing point depressant" is meant a compound that reduces the freezing point of a composition containing a β (1-3) β (1-4) glucan by, for example, about 1°C to 15°C, relative to that of the same composition lacking the freezing point depressant. The freezing point depressant should also act to substantially reduce or prevent the gellation of the composition containing the β (1-3) β (1-4) glucan.

The present invention provides a method of isolating a β (1-3) β (1-4) glucan from a milled cereal grain or a milled part of the cereal grain, as described in the claims. Also herein is described a method comprising:
(i) extracting the milled cereal grain or the milled part of the cereal grain with an alkaline solution to produce an extract containing at least about 0.4 weight percent β (1-3) β (1-4) glucan;
(ii) removing insoluble material, and removing particulate material having a particle size of greater than about 0.2 µm from the extract to produce a purified extract;
(iii) adding from about 10% to about 25% (w/w) of a C₁-C₄ alcohol to the purified extract to precipitate the β (1-3) β (1-4) glucan, and
(iv) isolating the β(1-3) β(1-4) glucan.

Cereal β-glucan can be isolated according to the purification method of the present invention from a milled whole cereal grain or a milled part of the cereal grain, such as the milled bran of the cereal grain. Preferably, the bran of the cereal grain is used. The cereal grain, or a part thereof that is extracted may be in the form of a coarsely milled meal or finely milled flour. The cereals that can be used in the present invention include, without limitation, any one of the cultivars of barley, oat, wheat, rye, corn, sorghum, and millet.

In the first step of the purification method of the present description, the milled grain or the milled part of the grain is slurried with reverse osmosis (RO) purified or deionized (DI) water to a final solids concentration of about 4 to about 10%, or about 6 to about 8%.

The pH value of the water used in the first step of the purification method can be from about 9.00 to about 10.00, more particularly, from about 9.25 to about 9.75, or from about 9.30 to about 9.50, and can be adjusted using an inorganic base, such as sodium hydroxide or potassium hydroxide. In one example, potassium hydroxide is used at a concentration from about 28 mM to about 35 mM. The use of a solution having a value of pH of between 9-10 generally reduces the amount of non-glucan polysaccharides and protein that is extracted during the first step, and, therefore, provides selective extraction of high molecule weight cereal β glucan molecules.

The extraction of the cereal β-glucan can be carried out over a 15 to 45 minute period, or over a 15-30 minute period. It is to be appreciated, however, that longer or shorter periods of extraction may be used depending on the type of cereal β-glucan used.

In the second step of the purification process, any insoluble material that cannot be extracted, for example hemicelluloses or lignins, is removed. Examples of methods that can be used to separate the insoluble material include, without limitation, centrifugation, preferably with a decanter centrifuge, and vibrating screening.

Any fine particulate material including some protein-based material is also removed from the alkaline solution in the second step of the method of the present invention. This material can be removed by adding an external flocculant or coagulant, or both. The flocculants or coagulants that can be used in the second step can have either a net positive, negative, or neutral charge. The coagulation step may be repeated one, or more than one time.

Examples of flocculants that can be used include, without limitation synthetic flocculants, such as polyacrylamides, quaternary acrylate salts and natural flocculant macromolecules such as chitosan, a natural polymer derived from chitin. Particular examples of flocculants include Tramfloc^{®} (Tramfloc Inc.), the cationic flocculant SURFLOC^{®} 34030 (Jes-Chem Ltd.), polyacrylamide (PAM) flocculants such as an Aquamark^{®} AQ 600 Series flocculant, or a SuperFloc^{®} C-500 Series flocculant (QEMI Inc.).

Examples of coagulants that can be used in the method of the present invention include, without limitation, inorganic electrolytes, such as alum, lime, ferric chloride, and ferrous sulfate, organic polymers, synthetic polyelectrolytes with anionic or cationic functional groups, and polyacrylamides.

The flocculants, coagulants, or a mixture thereof, may be used at a concentration of from about 0.09% to about 0.20% (w/vol), or from about 0.10% to about 0.13% (w/vol).

The alkaline solution may be incubated with the flocculant or coagulant for about 10 to about 40 minutes, or from about 10 to about 20 minutes at a temperature of from about 20 to about 40°C, or from about 20 to about 30°C. It is to be appreciated, however, that longer or shorter periods of time can be used to effect coagulation of the particulate material.

If negatively charged materials are to be removed from the solution containing the cereal β-glucan, then it is preferred that the flocculant or coagulant be positively charged. If positively charged materials are to be removed from the solution, then a negatively-charged flocculant or coagulant is preferred.

Without wishing to be bound by theory, the flocculants and coagulants that can be used in the method of the present invention function by forming large, dense aggregates with fine particulate matter, which can be easily separated from the aqueous solution containing the cereal β-glucan.

The coagulated material may be removed by centrifugation, using, for example, a disk-stack centrifuge. Other physical separation methods known to those of skill in the art can also be used to effect the separation of the coagulated material.

In the second step, any starch or related material that is present may be digested using an enzyme, such as, but not limited to an amylase. The enzyme may be used at a concentration of from about 0.05% to about 0.20% (vol/vol), from about 0.09% to about 0.15% (vol/vol), or from about 0.09% to about 0.11% (vol/vol). If an amylase is used, it is preferred that the alkaline solution be brought to an approximately neutral value of pH (i.e. ~pH 7) before adding the amylase. In an example, the solution containing the amylase is heated to a temperature of from about 50°C to about 100°C, or from about 70°C to about 90°C for about 20 to about 30 minutes to gelatinize the starch. The amylase will hydrolyse the starch and any related material. Generally, the amylase that is chosen to break down the starch material should be functional and stable within the temperature ranges indicated above. It is particularly preferred that the amylase not require a calcium co-factor to digest the starch material. Examples of such an amylase, include, without limitation, Termamyl^{®} LC (Novozymes A/S), and Spezyme^{®} FRED (Genencor International Inc.).

The completion of the hydrolysis reaction is determined when a sample withdrawn from the solution no longer produces a positive iodine test. At this point, the enzyme may be inactivated, by, for example, reducing the pH to a value of about 3.5 to about 4.0. The pH of the solution can be reduced using strong inorganic acids, such as hydrochloric acid or weak organic acids, such as malic acid or citric acid. It is preferred, however, that a strong inorganic acid, such as hydrochloric acid be used. In addition, it is preferred that the temperature of the solution be raised to between 85-90°C to denature protein present in solution.

The resulting acidified solution can then be filtered to remove any particulates and microbiological contaminants, through a filter pad that preferably has a cutoff point of about 20 µm. This filter may be coated with a pre-eoat of a filter aid having a thickness of about 2 to about 5 mm, such as Celite^{®} C65 (World Minerals), which has a nominal porosity of about 0.2 µm. An equivalent weight of a filter-aid, for example, an acid-washed pharmaceutical grade filter-aid, such as Celite^{®} C300 (World Minerals), may also be added as a body feed to the acidified solution prior to conducting the filtration step.

The filtration can be conducted using any one of a number of filtration devices. One particular example of a filtration device that can be used is a filter-press. In the case where the particle size of the material contained in the extract is less than 0.5 microns, then ceramic microfiltration and ultrafiltration can alternatively be used to filter the acidified solution.

In the third step of the purification method, the cereal β-glucan is precipitated from solution by adding a C₁-C₄ alcohol. The alcohol used to precipitate out the cereal β-glucan may be selected from the group consisting of methanol, ethanol, and isopropanol. If the cereal β-glucan isolated according to the procedure of,the present invention is to be used in the preparation of a pharmaceutical, or an edible product, then it is preferred that ethanol or isopropanol, more preferably, ethanol, be used.

As the concentration of the alcohol in the solution is increased, the cereal β-glucan is precipitated out as a fine colloidal suspension. The total amount of alcohol that is required to carry out the precipitation step may depend on the concentration of cereal β-glucan in solution. The alcohol is added to a final concentration of about 10% to about 25% by volume, preferably from about 15% to about 17% by volume. The present invention, therefore, avoids the use of high concentrations of alcohol (i.e. concentrations of greater than 50% by volume), which can cause severe dehydration of the cereal β**-**glucan and result in the need for homogenizers to disperse the cereal β-glucan.

It is preferred that the precipitation step be conducted at a low temperature, such as from about 1°C to about 10°C, preferably from about 1°C to about 5°C. In addition, it is preferred that the alcohol used in the precipitation step be cooled to a temperature of at least about -20°C before adding it to the β(1-3) β(1-4) glucan solution.

The final isolated cereal β-glucan material is a microdispersion or a nanodispersion, which is free of large particulates, and does not require additional filtration. Aqueous solutions of the cereal β-glucan isolated according to the present invention remain homogeneous after more than a year of being prepared.

Centrifugation using, for example, a disk-stack centrifuge, or a hydrocyclone can be used to isolate the suspended cereal β-glucan. If desired, the isolated β-glucan can be re-dissolved in an aqueous solution and re-precipitated with the C₁-C₄ alcohol to increase the purity of the β-glucan. The isolated solid can then be dried to a powder using, for example, vacuum drying, spray drying or drum drying. The preferred method of drying is vacuum drying, which produces a coarsely granular solid that can be further milled to a desired particle size, for example by hammer, pin or jet-milling. Vacuum drying, however, requires less heat, and can produce a relatively purer cereal β-glucan since Maillard and other by-products are minimized.

To prevent gellation of the cereal β-glucan at each of the steps of the purification method of the present invention, it is preferred that the addition of salts be minimized throughout the process. For example, it is preferred that reverse osmosis (RO) purified or deionized (DI) water be used, as well as an amylase not requiring a calcium cofactor, such as Termamyl^{®} LC (Novozymes A/S).

Without wishing to be bound by theory, one way in which gellation of solutions of cereal β-glucan can take place is by cross-linking of the molecules of cereal β-glucan, which is initiated by coordination of the cereal β-glucan molecules to ions, such as calcium. By using low amounts of salt throughout the process, cross-linking of cereal β-glucan molecules in the intermediate solutions formed in the method of the present invention, can, therefore, be minimized. In addition, by limiting the amount of salt introduced throughout the method of the present invention, the cereal β-glucan can be isolated essentially free of salts in the final step of the method.

The cereal β-glucan composition prepared by the purification method of the present invention generally has a purity of at least about 75%, and contains less than 10% ash impurities, less than 10% protein impurities, and less than 5% lipid impurities. More particularly, the cereal β-glucan composition produced by the methods of the present invention has a purity of at least about 92%, and contains less than 3.5% ash impurities, less than 3.5% protein impurities, and less than 1% lipid impurities. The yield of cereal β-glucan prepared according to the purification method of the present invention is generally from about 70 to about 72%.

Homogeneous solutions of the precipitated cereal β-glucan can be prepared by suspending the cereal β-glucan in reverse osmosis treated or deionized water at a temperature of about 30°C to about 45°C for a period of about 20 to about 30 minutes, or until most of the cereal β-glucan has been solubilized. The solution may then be pasteurized and a preservative added.

Aqueous solutions containing 1% cereal β-glucan, isolated according to the method of the present invention, generally have the following characteristics:
● a viscosity of about 200 to about 1500 cP, more particularly about 1000 to about 1500.cP.
● a clarity value of about 5 to about 100 NTU (Nominal Turbidity Units), more particularly about 5 to about 40 NTU.
● an ash concentration of about 0.02% to about 0.2%, more particularly about 0.02% to about 0.07%.
● a protein concentration of about 0.02% to about 0.2%, more particularly about 0.02% to about 0.07%.
● a lipid concentration of about 0.005% to about 0.1%, more particularly about 0.005% to about 0.02%.

Stabilized solutions of cereal β-glucan isolated according to the method of the present invention can be prepared in the manner described in U.S. Patent No. 6,284,886. If these solutions are to be used in the preparation of confectionery, pharmaceutical, or other related compositions, then the preservatives used in the method described in U.S. Patent No. 6,284,886 should be one that is approved for human consumption and pharmaceutical use, such as, but not limited to potassium sorbate, sorbic acid, benzalkonium chloride, and parabens.

The cereal β-glucan isolated according to the method of the present invention is of particular use in wound healing, and in reducing wrinkles, where transfer of cereal β-glucan across intact skin, can lead to the rebuilding of collagen through the stimulation of fibroblast growth.

In a further aspect of the present description, there is provided a pharmaceutical composition comprising:
an effective amount of a β (1-3) β (1-4) glucan, and
an effective amount of a botanical extract, or a pharmaceutically active agent.

The β (1-3) β (1-4) glucan used in the above-defined pharmaceutical composition may be prepared according to the isolation methods which have been described above. The β (1-3) β (1-4) glucan of the above-defined pharmaceutical compositions may be derived from a cereal grain or a part of the cereal grain. In an example, the cereal is selected from the group consisting of a cultivar of barley, a cultivar of oat, a cultivar of wheat, a cultivar of rye, a cultivar of sorghum, a cultivar of millet, a cultivar of corn, and a mixture thereof.

The β (1-3) β (1-4) glucan used in the pharmaceutical compositions of the present description may be a β (1-3) β (1-4) glucan composition having a purity of at least about 75%, and containing less than 10% ash impurities, less than 10% protein impurities, and less than 5% lipid impurities. More particularly, the present description relates to a pharmaceutical composition comprising a β (1-3) β (1-4) glucan composition having a purity of at least about 92%, and containing less than 3.5% ash impurities, less than 3.5 % protein impurities, and less than 1% lipid impurities.

The compositions of the present description can be formed by mixing an aqueous solution comprising about 0.01 wt. % to about 20 wt. %, about 0.01 wt. % to about 1.2% wt. %, about 0.1 wt. % to about 1.1 wt. %, or about 0:5 wt. % to about 1 wt. % of the β (1-3) β (1-4) glucan with one, or more than one compound of interest, such as a botanical extract, or a pharmaceutically active agent. The one, or more than one compound of interest may be present in an amount of from about 0.01 wt. % to about 40 wt. %, from about 0.01 wt. % to about 25 wt. %, from about 0.01 wt% to about 4 wt. %, from about 0.1 wt. % to about 1.4 wt. %, or from about 0.5 wt. % to about 1.2 wt. %. It is preferred that the resulting compositions be left undisturbed after being mixed for a period of time sufficient to allow the formation of a homogeneous composition in the form of a suspension, emulsion or gel. In many cases, the amount of time required to obtain a homogeneous composition is from about eight to about 16 hours. It is to be appreciated, however, that shorter or longer periods of time may be required, depending on the quantity and purity of the β (1-3) β (1-4) glucan used, as well as on the quantity and nature of each of the one, or more than one compound of interest Compositions of the present description which are in the form of a gel may be converted into a more fluid state by gentle agitation.

Without wishing to be bound by theory, the formation of the homogeneous suspension, emulsion or gel is believed to be caused by the one, or more than one compound of interest being sequestered or encapsulated within the β (1-3) β (1-4) glucan, and by the subsequent formation of hydrogen bonds between molecules of the one, or more than one compound of interest and the β (1-3) β (1-4) glucan. Another possibility is that the P (1-3) β (1-4) glucan acts as a surfactact or emulsifying agent by reducing the interfacial tension at the boundaries between the one, or more than one compound of interest and the aqueous phase within which the β (1-3) β (1-4) glucan is dispersed, and, consequently, effectively solubilizes the one, or more than one compound within the aqueous phase.

The beta glucan solutions used in preparing the compositions of the present description are generally prepared from a beta glucan having a purity of from about 65% to about 100%, from about 75% to about 100%, or from about 85% to about 100%. In particular, beta glucan solutions used in preparing the compositions of the present description generally contain less than 20%, more particularly less than 15%, even more particularly less than 10%, most particularly less than 5% of impurities, such as protein, lipid, carbohydrate, and particulate impurities.

Examples of the botanical extract that may be used in the pharmaceutical compositions according to the present description include, without limitation, extracts of Guarana, *Ginkgo biloba,* Kola nut, Goldenseal, Golo Kola, *Schizandra,* Elderberry, St. John's Wort, Valerian and *Ephedra,* black tea, white tea, java tea, garlic oil, fiber, green tea, lemon oil, mace, licorice, onion oil, orange oil, rosemary, milk thistle, *Echinacea,* Siberian ginseng or *Panax ginseng,* lemon balm, *Kava kava,* matte, bilberry, soy, grapefruit, seaweed, hawthorn, lime blossom, sage, clove, basil, curcumin, taurine, wild oat herb, oat grain, dandelion, gentian, aloe vera, hops, cinnamon, peppermint, grape, chamomile, fennel, marshmallow, ginger, slippery elm, cardamon, coriander, anise, thyme, rehmannia, eucalyptus, menthol, schisandra, withania, cowslip, lycium, and passion flower.

In a particular example, the botanical extract is an extract of an oat grain. More particularly, the botanical extract is an oat grain extract, which contains avenanthramide.

As an example of a pharmaceutically active agent there is mentioned an antihistamine, a decongestant, a corticosteroid, a non-steroidal anti-inflammatory drug, a bronchodilator, a vasodilator, or a local anaesthetic.

Other examples of the pharmaceutically active agent botanical extract that may be used in the pharmaceutical compositions of the present description include, without limitation, beta sitosterol, caffeine, cafestol, D-limonene, kabweol, nomilin, oltipraz, sulphoraphane, tangeretin, folic acid, and menthol.

The pharmaceutical composition of the present description can be used in the form of a spray, a liquid, which may in the form of drops, or a gel. In an example, the botanical extract, and the pharmaceutically active agent comprises compounds that are readily absorbed through the mucosa of the oral cavity, the mucosa of the nasal cavity, or through gum tissue.

It is preferred that the pharmaceutical compositions of the present description containing an anesthetic be applied to a specific, localized region of the gums or a surface of the oral cavity of a subject. It is also preferred that the compositions of the present description, which contain a vasodilating agent, such as nitroglycerin, be applied underneath the tongue of a subject. The pharmaceutical compositions of the present description, which comprise an antihistamine, a decongestant, a corticosteroid, or a non-steroidal anti-inflammatory drug can be applied to the back of the oral cavity, or to the nasal cavity of a subject to allow medication released from the composition to be inhaled by the subject. Pharmaceutical compositions according to the present description, which comprise a consumable botanical extract, may be used as a mouthrinse and expectorated after being used, or, alternatively, may be swallowed.

The pharmaceutical compositions of the present description may contain a pharmaceutically acceptable diluent or carrier, which is chosen based on the intended route of administration and standard pharmaceutical practice.

The pharmaceutical compositions of the present description may also be administered orally in the form of tablets or capsules containing excipients, such as starch or lactose, or in the form of elixirs or suspensions containing flavoring or coloring agents. They may be injected parenterally, for example, intravenously, intramuscularly or subcutaneously. For parenteral administration, they are best used in the form of an isotonic sterile aqueous solution.

The pharmaceutical compositions of the present description may also be administered, topically when treating inflammatory conditions of the skin in the form of a cream, a jelly, a gel, a paste, or an ointment. For example, pharmaceutical compositions of the present description, which contain a corticosteroid, a non-steroidal anti-inflammatory drug, or a botanical extract may be used as a topical composition, in the form of a cream.

Example 2 demonstrates that β (1-3) β (1-4) glucan prepared according to the method of the present invention, and applied in the form of a topical composition to the surface of a section of skin, can significantly cross into the horny layer, the epidermis, the dermis and the subcutis layers of the skin. These results suggest that a pharmaceutically active agent or a botanical extract encapsulated by the β (1-3) β (1-4) glucan isolated according to the present invention could also be effectively transferred down to the dermis and subcutis layers of the skin of a subject.

According the third aspect of the present description relates a composition comprising a β (1-3) β (1-4) glucan and from about 1% to about 40% by weight of a freezing point depressant. In other examples, the freezing point depressant is present in an amount of from about 1% to about 30% by weight, or from about 10% to about 20% by weight.

The β (1-3) β (1-4) glucan used in the composition of the third aspect of the present description can be prepared according to the isolation methods of the present invention. Other cereal beta glucans that can be used in this composition include those available from commercial suppliers, such as Sigma Chemical Co. (St. Louis, MO) and Ceapro Inc. (Edmonton, AB, Canada).

In an example of the composition of the third aspect of the present the β (1-3) β (1-4) is present in an amount of from about 1.2% to about 1.6% or from about 1.2% to about 1.3% by weight. In another example, the freezing point depressant is selected from the group consisting of glycerol, propylene glycol, butylene glycol and pentylene glycol.

In a further example of the composition of the third aspect of the present description the β (1-3) β (1-4) glucan is a β (1-3) β (1-4) glucan composition having a purity of at least about 75%, and containing less than 10% ash impurities, less than 10% protein impurities, and less than 5% lipid impurities. More particularly, the β - glucan composition has a purity of at least about 92%, and contains less than 3.5% ash impurities, less than 3.5 % protein impurities, and less than 1% lipid impurities. The β (1-3) β (1-4) glucan may also have a clarity value of about 5 to about 100 NTU.

The cereal β-glucan content can be determined using a number of methods, known to those skilled in the art (McCleary AOAC method). For example, cereal β-glucan content can be assessed colorimetrically and/or by standard analytical techniques such as size exclusion chromatography and HPLC (see Wood et al. Cereal 'Chem. (1977) 54:524; Wood et al. Cereal Chem. (1991) 68:31-39; and Wood et al. Cereal Chem. (1991) 68:530-536). β-glucans can also be analyzed enzymatically using commercially available kits, such as Megazyme (Ireland) employing the techniques of McCleary and Glennie-Holmes J. Inst. Brew. (1985) 91:285.

Viscosities can be measured with a rotational, shear-type viscometer such as the Brookfield Syncro-Lectric or the Haake Rotovisco. Methods of using the instrument are known to those skilled in the art. Routinely, measurements are made at four speeds of disc rotation at a constant temperature of 25°C.

The following example is provided to exemplify the present invention. Variations and alterations will be readily apparent to those skilled in the art.

### Example 1: Method for purifying cereal β-glucan derived from oat bran

Oat bran (The Quaker Oats Company) was slurried with alkaline reverse osmosis (RO) water at a pH of about 9.5 to a final solids concentration of 4-10%. The temperature was maintained at 45°C ± 5°C. The cereal β-glucan was extracted from the oat bran over a period of 30 minutes. After this time, the solids were removed by centrifugation with a decanter centrifuge. The centrate was cooled to room temperature, and the cationic flocculant SURFLOC^{®} 34030 (Jes-Chem Ltd.) was added at a 0.2% concentration. Following an incubation period of 20 minutes, coagulated particulate material was removed by centrifugation using a disk-stack centrifuge. The pH of the centrate was adjusted to approximately neutral, heated to >72°C to gelatinize starch, and treated with the heat-stable amylase Termamyl^{®} LC (Novozymes A/S). When the solution no longer produced a positive iodine test, the pH was reduced to about 4.0 to inactivate the enzyme, and the mixture was heated to 85°C for 30 minutes to denature the protein present. The solution was cooled to 4°C for one hour, and then heated to a temperature of about 72°C. An equivalent weight of CELITE^{®} C300 (World Minerals) was added to the solution, and the mixture was then filtered using a filter-press containing 25 µm filter-papers and pre-coated to a depth of about 4 mm with CELITE^{®} C65 (World Minerals). The filter press was preheated to a temperature of about 65°C, and the pH of the feedstream for the filter press was adjusted to 4.5 before the β-glucan solution was filtered. After the β-glucan solution was passed through the filter, the press was flushed with reverse osmosis water resulting in a clear, pale yellow coloured β-glucan solution. The β-glucan solution was cooled to 5°C and 95% ethanol at a temperature of -20°C was added to a final volume of about 15% (w/w) with stirring. A suspension of β-glucan was formed that was immediately separated from the solution by centrifugation with a disk-stack centrifuge. The isolated solid β-glucan was added to RO water at 45°C, allowed to disperse and then heated to between 60-70°C to produce a clear colorless solution containing about 1% β-glucan. The separated β-glucan was colourless, had a purity of greater than 75%, a viscosity >500 cP, and an exception clarity <50 NTU, as measured using a turbidity meter.

### Example 2. Quantification of the Distribution of Purified β-Glucan Applied as an Aqueous Composition to Abdominal Skin Sections

Human abdominal skin was received under informed consent from five healthy donors having undergone plastic surgery. The skin from each patient was liberated from subcutaneous fat, and cut into three sections. The skin sections were frozen in liquid nitrogen and sterilized overnight with a dose of 25 kGy of gamma-radiation. The irradiated samples were each mounted in a 20 mL volume FRANZ-CELL^{®}-like perfusion chamber (PHACOCELL^{®}, PhaCos GmbH, D-82131-Gauting, Germany; see Artmann, C. W. In vitro percutaneous absorption into human skin, Fundam. Appl. Toxicol., 28, 1-5 (1996)) containing an acceptor medium. Using a microdose applicator, the irradiated samples of skin were coated with a 5 mg/cm² dosage of Composition 1455, Composition 1450 or a control composition. The Compositions 1455 and 1450 were aqueous compositions containing 5% and 50%, respectively, of the β (1-3) β (1-4) glucan prepared according to the isolation method of the present invention (see Example 1). The control composition was an aqueous composition that did not contain any β (1-3) β(1-4) glucan. The chamber was kept free of air bubbles while filling in order to ensure complete and even rinsing of the skin tissue. Pressure compensation, inside and outside of the chamber and a constant humidity of air was provided by ventilation. The skin temperature was monitored with temperature sensors, and the moisture content of the skin sections was monitored with a comeometer. The medium was regulated at 36°C and circulated continuously. Skin humidity was kept at about 65 corneometer units, and the skin surface temperature was kept at 32°C via a ventilation channel. The above conditions were maintained by regulation of the temperature of the medium by using a heating plate at the base of the chamber, and air tubes, and by adjusting the flow of air in the chamber. The skin sections were supplied by the uniformly circulating nutrient medium, which rinsed their lower surfaces. The area of application for all samples was fixed at 10 cm². The skin samples were incubated for eight hours under non-occlusive (open) conditions.

At the end of the incubation period, swab samples of the skin sections were taken with both dry cotton gauze swabs and cotton gauze swabs moistened with 0.2 mL of 70% methanol/H₂O. The skin sections were removed from the Phacocell^{®} chamber and immediately frozen in liquid nitrogen. The skin sections were then cut into 15 µm slices from the horny layer to the deeper dermis. The skin sections were allowed to air dry on clean glass slides and not fixed with any fluid. The slices were then stained with BACTIDROP™ Calcofluor White for 30 seconds and then washed of excess stain with deionized water. The staining and washing steps were repeated twice. The stained sample was covered with a clean glass cover slip and examined by fluorescence with a LEIKA^{®} fluorescent microscope having an exciter filter ranging between 400-500 nm with a peak of 440 nm, a barrier filter of 500-520 nm, and a xenon arc (burner) lamp. BACTIDROP™ Calcofluor White is a non-specific fluorochrome that binds to cellulose, and upon excitation with long wavelength ultraviolet light delineates the cell walls of cellulose-containing organisms. The deposition of the β-glucan molecules was monitored and quantified using bright fluorescence, focus inverted to white spots (3 - 5 µm) seen upon the cell walls of the samples and in the intercellular interstices.

The mean percent depositions as determined by the above fluorescence staining method are shown in Table 3. Significant fluorescent staining values (>5%) were observed in the horny layer and in the epidermis of the skin samples treated with Composition 1455 and Composition 1450. Relatively lower values were observed in the dermis and subcutis layers of the skin samples treated with Composition 1450 and Composition 1455. Fluorescence staining values of <1% were observed with the skin sections that were treated with the control composition. ,

**Table 3. Mean Percent Deposition of β (1-3) β (1-4) Glucan in Different Layers of Abdominal Skin**

| | Mean Percent Deposition | | | | | |
|---|---|---|---|---|---|---|
| | COMPOSITION 1455 | | COMPOSITION 1450 | | Control | |
| | Percent | Standard Deviation | Percent | Standard Deviation | Percent | Standard Deviation |
| Medium | - | - | - | - | - | - |
| Swab | - | - | - | - | - | - |
| Horny layer | 8.7 | 1.2 | 12.8 | 1.9 | 0.6 | 0.2 |
| Epidermis | 5.9 | 1.3 | 11.6 | 2.0 | 0.8 | 0.2 |
| Dermis | 2.4 | 0.5 | 4.1 | 1.1 | 0.6 | 0.1 |
| subcutis | 1.4 | 0.5 | 1.5 | 0.4 | 0.9 | 0.1 |

The documentation of the findings by photographs (not shown) also demonstrated a significant uptake of the β-glucan into the epidermis layer of the skin samples.

The measurement of fluorescence was performed in accordance with quality control procedures and documentations. Control numbers of the BACTIDROP™ Calcofluor White were tested using recognized quality control organisms and were found to be acceptable. (Microbiology M. Pettenkofer Institute, München). Statistical evaluation was carried out by the statistics software package SAS/STATISTICA^{®}. Both the hardware and the software used were validated.

The present invention has been described with regard to preferred embodiments. However, it will be obvious to persons skilled in the art that a number of variations and modifications can be made

## Claims

1. A method of isolating a β (1-3) β (1-4) glucan from a milled cereal grain or a milled part of the cereal grain, comprising:
(i) extracting the milled cereal grain or the milled part of the cereal grain with an alkaline solution having a value of pH of between 9 to 10 for a period of time of about 15 to about 45 minutes to produce an extract containing at least about 0.4 weight percent β (1-3) β (1-4) glucan;
(ii) removing insoluble material, and removing particulate material having a particle size of greater than about 0.2 µm from said extract to produce a purified extract;
(iii) adding from about 10% to about 25% (vol/vol) of a C₁-C₄ alcohol to the purified extract to precipitate the β (1-3) β (1-4) glucan, and
(iv) isolating the β (1-3) β (1-4) glucan.

2. The method of claim 1, wherein, in said step of adding (step iii), about 10% to about 20% (vol/vol) of an alcohol selected from the group consisting of methanol, ethanol and isopropanol, is used to precipitate the β (1-3)β (1-4) glucan from said purified extract.

3. The method of claim 2, wherein about 10% to about 20% (vol/vol) of ethanol is used to precipitate the β (1-3) β (1-4) glucan from said purified extract.

4. The method of claim 1, wherein, said step of removing particulate material further comprises:
one, or more than one step of adding a flocculant, a coagulant or both a flocculant and a coagulant to said extract to coagulate particulate material having a particle size of greater than about 0.2 µm, and removing coagulated material from said extract;
digesting starch material in said extract, and
filtering out particulate material having a particle size of greater than 0.2 µm from said extract to produce the purified extract.

5. The method of claim 4, wherein, in said step of digesting, said starch material is digested with an enzyme.

6. The method of claim 5, wherein prior to digesting said starch material, said alkaline solution is neutralized.

7. The method of claim 6, wherein following the digestion of said starch material, said enzyme is inactivated.

8. The method of claim 7, wherein said enzyme is inactivated by acidifying the neutralized solution.

9. The method of claim 5, wherein said enzyme is an amylase.

10. The method of claim 9, wherein said amylase does not require a calcium cofactor.

11. The method of claim 1, wherein the cereal is selected from the group consisting of a cultivar of barley, a cultivar of oat, a cultivar of wheat, a cultivar of rye, a cultivar of sorghum, a cultivar of millet, and a cultivar of corn.

12. The method of claim 1, wherein said step of adding (step iii) is conducted at a temperature of from about 1°C to about 10°C.

13. The method of claim 1, further comprising one, or more than one step of dissolving the isolated β (1-3) β (1-4) glucan in an aqueous solution, precipitating the β (1-3) β (1-4) glucan by adding between 10% to 25% (vol/vol) of the C₁-C₄ alcohol to the aqueous solution, and isolating the β (1-3) β (1-4) glucan.

14. The method of claim 4, wherein the flocculant is selected from the group consisting of a polyacrylamide, a quaternary acrylate salt and a natural flocculant macromolecule, and the coagulant is selected from the group consisting of alum, lime, ferric chloride, ferrous sulfate, an organic polymer and a synthetic polyelectrolyte with anionic or cationic functional groups.

15. The method of claim 1, wherein about 15% to about 17% (vol/vol) of the C₁-C₄ alcohol is added to the purified extract in step (iii).

16. The method of claim 1, wherein the milled cereal grain or the milled part of the cereal grain is extracted with an alkaline solution having a value of pH of about 9.25 to about 9.75.

17. The method according to claim 4, wherein the step of filtering out material having a particle size of greater than 0.2 µm from said extract is conducted using a filter coated with a pre-coat of a filter aid having a porosity of 0.2 µm.

## Patentansprüche

1. Verfahren zum Isolieren eines β(1-3)-β(1-4)-Glukans aus einem gemahlenen Getreidekorn oder einem gemahlenen Teil des Getreidekorns, das Folgendes umfasst:
(i) Extrahieren des gemahlenen Getreidekorns oder des gemahlenen Teils des Getreidekorns mit einer alkalischen Lösung mit einem pH-Wert zwischen 9 und 10 über einen Zeitraum von ungefähr 15 bis ungefähr 45 Minuten, wodurch man zu einem Extrakt gelangt, der mindestens ungefähr 0,4 Gewichtsprozent β(1-3)-β(1-4)-Glukan enthält;
(ii) Entfernen von unlöslichem Material und Entfernen von teilchenförmigem Material mit einer Teilchengröße von mehr als ungefähr 0,2 µm aus dem Extrakt, wodurch man zu einem aufgereinigten Extrakt gelangt;
(iii) Versetzen des aufgereinigten Extrakts mit ungefähr 10% bis ungefähr 25% (vol/vol) eines C₁-C₄-Alkohols, wodurch das β(1-3)-β(1-4)-Glukan ausgefällt wird, und
(iv) Isolieren des β(1-3)-β(1-4)-Glukans.

2. Verfahren nach Anspruch 1, wobei in dem Schritt des Versetzens (Schritt iii) ungefähr 10% bis ungefähr 20% (vol/vol) eines Alkohols, ausgewählt aus der Gruppe bestehend aus Methanol, Ethanol und Isopropanol, eingesetzt wird, um das β(1-3)-β(1-4)-Glukan aus dem aufgereinigten Extrakt auszufällen.

3. Verfahren nach Anspruch 2, wobei ungefähr 10% bis ungefähr 20% (vol/vol) Ethanol eingesetzt wird, um das β(1-3)-β(1-4)-Glukan aus dem aufgereinigten Extrakt auszufällen.

4. Verfahren nach Anspruch 1, wobei der Schritt des Entfernens von teilchenförmigem Material weiterhin Folgendes umfasst:
einen oder mehr als einen Schritt des Versetzens des Extrakts mit einem Ausflockungsmittel, einem Koagulationsmittel oder sowohl einem Ausflockungsmittel als auch einem Koagulationsmittel, um teilchenförmiges Material mit einer Partikelgröße von mehr als ungefähr 0,2 µm zu koagulieren und das koagulierte Material aus dem Extrakt zu entfernen;
Verdauen von Stärkematerial in dem Extrakt, und
Abfiltrieren von teilchenförmigem Material mit einer Teilchengröße von mehr als 0,2 µm von dem Extrakt, wodurch man zu dem aufgereinigten Extrakt gelangt.

5. Verfahren nach Anspruch 4, wobei in dem Schritt des Verdauens das Stärkematerial mit einem Enzym verdaut wird.

6. Verfahren nach Anspruch 5, wobei die alkalische Lösung vor dem Verdauen des Stärkematerials neutralisiert wird.

7. Verfahren nach Anspruch 6, wobei das Enzym nach dem Verdauen des Stärkematerials inaktiviert wird.

8. Verfahren nach Anspruch 7, wobei das Enzym durch Ansäuern der neutralisierten Lösung inaktiviert wird.

9. Verfahren nach Anspruch 5, wobei es sich bei dem Enzym um eine Amylase handelt.

10. Verfahren nach Anspruch 9, wobei die Amylase keinen Calcium-Cofaktor benötigt.

11. Verfahren nach Anspruch 1, wobei das Getreide aus der Gruppe bestehend aus einer Gerstensorte, einer Hafersorte, einer Weizensorte, einer Roggensorte, einer Sorghumsorte, einer Hirsesorte und einer Maissorte ausgewählt ist.

12. Verfahren nach Anspruch 1, wobei der Schritt des Versetzens (Schritt iii) bei einer Temperatur von ungefähr 1°C bis ungefähr 10°C durchgeführt wird.

13. Verfahren nach Anspruch 1, das weiterhin einen oder mehr als einen Schritt des Auflösens des isolierten β(1-3)-β(1-4)-Glukans in einer wässrigen Lösung, Ausfällen des β(1-3)-β(1-4)-Glukans durch Versetzen der wässrigen Lösung mit 10% bis 25% (vol/vol) des C₁-C₄-Alkohols und Isolieren des β(1-3)-β(1-4)-Glukans umfasst.

14. Verfahren nach Anspruch 4, wobei das Ausflockungsmittel aus der Gruppe bestehend aus einem Polyacrylamid, einem quartären Acrylatsalz und einem natürlichen makromolekularem Ausflockungsmittel ausgewählt ist und das Koagulationsmittel aus der Gruppe bestehend aus Alaun, Kalk, Eisen(III)-chlorid, Eisen(II)-sulfat, einem organischen Polymer und einem synthetischen Polyelektrolyten mit anionischen oder kationischen funktionellen Gruppen ausgewählt ist.

15. Verfahren nach Anspruch 1, wobei der aufgereinigte Extrakt in Schritt (iii) mit ungefähr 15% bis ungefähr 17% (vol/vol) des C₁-C₄-Alkohls versetzt wird.

16. Verfahren nach Anspruch 1, wobei das gemahlene Getreidekorn oder der gemahlene Teil des Getreidekorns mit einer alkalischen Lösung mit einem pH-Wert von ungefähr 9,25 bis ungefähr 9,75 extrahiert wird.

17. Verfahren nach Anspruch 4, wobei der Schritt des Abfiltrierens von Material mit einer Partikelgröße von mehr als 0,2 µm von dem Extrakt mit einem Filter, das mit einem Precoat einer Filtrierhilfe mit einer Porosität von 0,2 µm beschichtet ist, durchgeführt wird.

## Revendications

1. Procédé d'isolement d'un β(1-3)β(1-4) glucane à partir d'un grain de céréale moulu ou d'une partie moulue du grain de céréale comprenant :
(i) l'extraction du grain de céréale moulu ou de la partie moulue du grain de céréale avec une solution alcaline ayant un pH compris entre 9 et 10 pendant une durée de temps d'environ 15 à environ 45 minutes pour produire un extrait contenant au moins environ 0,4 % en poids de β(1-3)β(1-4) glucane ;
(ii) l'élimination de la matière insoluble, et l'élimination de la matière particulaire ayant une taille de particule supérieure à environ 0,2 µm dudit extrait pour produire un extrait purifié ;
(iii) l'addition d'environ 10 % à environ 25 % (vol/vol) d'un alcool en C₁-C₄ à l'extrait purifié pour précipiter le β(1-3)β(1-4) glucane, et
(iv) l'isolement du β(1-3)β(1-4) glucane.

2. Procédé selon la revendication 1, **caractérisé en ce que**, dans ladite étape d'addition (étape iii), on utilise d'environ 10 % à environ 20 % (vol/vol) d'un alcool choisi dans le groupe constitué par le méthanol, l'éthanol et l'isopropanol pour précipiter le β(1-3)β(1-4) glucane dans ledit extrait purifié.

3. Procédé selon la revendication 2, **caractérisé en ce qu'**on utilise d'environ 10 % à environ 20 % (vol/vol) d'éthanol pour précipiter le β(1-3)β(1-4) glucane dans ledit extrait purifié.

4. Procédé selon la revendication 1, **caractérisé en ce que** ladite étape d'élimination de matière particulaire comprend en outre :
une ou plusieurs étapes d'addition d'un floculant, d'un coagulant ou à la fois d'un floculant et d'un coagulant audit extrait pour coaguler la matière particulaire ayant une taille de particule supérieure à environ 0,2 µm, et l'élimination de la matière coagulée dudit extrait ;
la digestion de la matière amylacée dans ledit extrait, et
la filtration de la matière particulaire ayant une taille de particule supérieure à 0,2 µm dudit extrait pour produire l'extrait purifié.

5. Procédé selon la revendication 4, **caractérisé en ce que** dans ladite étape de digestion, ladite matière amylacée est digérée par une enzyme.

6. Procédé selon la revendication 5, **caractérisé en ce qu'**avant la digestion de ladite matière amylacée, ladite solution alcaline est neutralisée.

7. Procédé selon la revendication 6, **caractérisé en ce qu'**après la digestion de ladite matière amylacée, ladite enzyme est inactivée.

8. Procédé selon la revendication 7, **caractérisé en ce que** ladite enzyme est inactivée par acidification de la solution neutralisée.

9. Procédé selon la revendication 5, **caractérisé en ce que** ladite enzyme est une amylase.

10. Procédé selon la revendication 9, **caractérisé en ce que** ladite amylase ne nécessite pas de cofacteur calcium.

11. Procédé selon la revendication 1, **caractérisé en ce que** la céréale est choisie dans le groupe constitué par un cultivar d'orge, un cultivar d'avoine, un cultivar de blé, un cultivar de seigle, un cultivar de sorgho, un cultivar de millet, et un cultivar de maïs.

12. Procédé selon la revendication 1, **caractérisé en ce que** ladite étape d'addition (étape iii) est effectuée par une température d'environ 1°C à environ 10°C.

13. Procédé selon la revendication 1, comprenant en outre une ou plusieurs étapes de dissolution du β(1-3) β (1-4) glucane isolé dans une solution aqueuse, de précipitation du β(1-3)β(1-4) glucane en ajoutant entre 10 % et 25 % (vol/vol) de l'alcool en C₁-C₄ à la solution aqueuse, et d'isolement du β (1-3) β (1-4) glucane.

14. Procédé selon la revendication 4, **caractérisé en ce que** le floculant est choisi dans le groupe constitué par un polyacrylamide, un sel d'acrylate quaternaire et une macromolécule floculante naturelle, et le coagulant est choisi dans le groupe constitué par l'alun, la chaux, le chlorure ferrique, le sulfate ferreux, un polymère organique et un polyélectrolyte synthétique ayant des groupes fonctionnels anioniques ou cationiques.

15. Procédé selon la revendication 1, **caractérisé en ce qu'**on ajoute à l'extrait purifié dans l'étape (iii) d'environ 15 % à environ 17 % (vol/vol) de l'alcool en C₁-C₄.

16. Procédé selon la revendication 1, **caractérisé en ce que** le grain de céréale moulu ou la partie moulue du grain de céréale sont extraits avec une solution alcaline ayant un pH d'environ 9,25 à environ 9,75.

17. Procédé selon la revendication 4, **caractérisé en ce que** l'étape de filtration de matière ayant une taille de particule supérieure à 0,2 µm dudit extrait est effectuée en utilisant un filtre revêtu d'une pre-couche d'adjuvant de filtration ayant une porosité de 0, 2 µm.
